# EUROPEAN PATENT SPECIFICATION

(11) **EP 2 167 343 B1**
(45) Date of publication and mention of the grant of the patent: **29.12.2010**
(21) Application number: 08789096.8
(22) Date of filing: 09.07.2008
(51) Int. Cl.: B60K 28/06, G01N 33/497, G01N 33/98

(54) **BREATHALYZER TEST DEVICE**
ALCOTESTGERÄT
DISPOSITIF D'ALCOOTEST

(30) Priority: 10.07.2007 JP 2007181145
(43) Date of publication of application: 31.03.2010
(73) Proprietor: Toyota Jidosha Kabushiki Kaisha, Toyota-shi, Aichi-ken 471-8571 (JP)
(72) Inventor: OZAKI, Osamu, Toyota-shi Aichi-ken 471-8571 (JP); WATANABE, Atsushi, Toyota-shi Aichi-ken 471-8571 (JP)
(74) Representative: Albutt, Anthony John
(86) International application number: PCT/IB2008/002150
(87) International publication number: WO 2009/007844

(56) References cited:
- ES-A1- 2 172 447
- US-A- 3 890 826
- US-A1- 2006 237 252
- US-B1- 6 927 694

## Description

### BACKGROUND OF THE INVENTION

### 1. Field of the Invention

The present invention relates to a breathalyzer test device.

### 2. Description of the Related Art

Following alcohol consumption, a person's judgment, attentiveness, motor ability and so on deteriorate. Therefore, when a person drives a vehicle under the influence of alcohol, danger increases, leading to an increased risk of accident. However, drivers who drink and drive do exist, and therefore various devices for suppressing drunk driving have been developed. For example, in a remote monitoring control system described in Japanese Patent Application Publication No. 2004-148950 (JP-A-2004-148950), a drive is urged to perform an alcohol concentration check by a warning, and when the alcohol concentration exceeds a fixed value, the driver is urged to stop driving by a warning and an alarm. When the driver ignores the warning and so on and continues to driver, the driver is again urged to stop driving by the warning and the alarm, and moreover, it is possible to stop the vehicle engine. Further, when an alcohol concentration check has not been performed for a fixed time period or longer following the warning, the driver is warned again.

US 3 890 826 A1 discloses an intoxicated driver capability tester comprising a detecting device to detect the amount of alcohol in a driver's body and a device to decide the capability of driver, whereby in case that the amount of alcohol in a driver's body is detected to be less than a predetermined value, the tester permits the engine to be started without deciding the capability of the driver for driving.

In a conventional drunk driving suppression device, the driver is urged to take a breathalyzer test periodically (at fixed time intervals while driving, for example), and therefore the breathalyzer test is extremely troublesome and a burden to a driver who has not consumed any alcohol. Furthermore, the accuracy of the breathalyzer test is not 100%, and therefore alcohol consumption may be detected erroneously even though the driver has not consumed alcohol. If the vehicle is immobilized in this case, the driver is unable to release the immobilization and cannot operate the vehicle.

### SUMMARY OF THE INVENTION

Therefore, the present invention provides a breathalyzer test device that reduces the above-described troublesomeness involved in a breathalyzer test.

A breathalyzer test device according to an aspect of the present invention includes: vigilance detecting means for detecting a vigilance of a test subject; and breathalyzer test means for performing a breathalyzer test on the test subject based on the vigilance detected by the vigilance detecting means.

In this breathalyzer test device, the vigilance of the test subject is detected by the vigilance detecting means. Following alcohol consumption, vigilance typically deteriorates, and therefore a cause-and-effect relationship exists between alcohol consumption and vigilance. Accordingly, when vigilance is used, a breathalyzer test may be performed as required (when there is a high possibility that alcohol has been consumed). Hence, in the breathalyzer test device, the breathalyzer test is performed on the test subject by the breathalyzer test means based on vigilance. By taking the vigilance of the test subject into account in this manner, the troublesomeness of the breathalyzer test for the test subject may be reduced in the breathalyzer test device, thereby easing the burden on the test subject. Moreover, a breathalyzer test may be performed when the possibility of alcohol consumption is determined based on the vigilance, and therefore the accuracy of the test may be improved and erroneous detections may be suppressed.

In the breathalyzer test device according to the aspect described above, the breathalyzer test means may include implementation determining means for determining whether or not to perform the breathalyzer test based on the vigilance detected by the vigilance detecting means, and the breathalyzer test may be performed only when the implementation determining means determines that the breathalyzer test is to be performed.

Following alcohol consumption, vigilance deteriorates below normal levels, and therefore, when vigilance is normal (i.e. when vigilance is high), there is no need to perform a breathalyzer test, whereas when vigilance deteriorates below normal levels (i.e. when vigilance is low), it becomes necessary to perform the breathalyzer test. Hence, when the vigilance is detected by the vigilance detecting means in the breathalyzer test device according to the present invention, the implementation determining means determines whether or not to perform a breathalyzer test based on the vigilance, and the breathalyzer test is implemented by the breathalyzer test device only when the implementation determining means determines that the breathalyzer test is to be performed. Thus, the breathalyzer test is realized that is performed only when there is a possibility that alcohol has been consumed and a breathalyzer test is required.

In the breathalyzer test device according to the aspect described above, the vigilance detecting means may detect the vigilance based on an eye closure state of the test subject. As vigilance deteriorates (as sleepiness increases), a person's eyes gradually close until s/he falls asleep. Hence, vigilance may be detected based on the eye closure state. It is particularly inconceivable that the driver of a vehicle would drive with his/her eyes closed, and therefore it is highly likely that vigilance has deteriorated when the eyes are closed.

In the breathalyzer test device according to the aspect described above, the vigilance detecting means may include image capturing means for capturing an image including an eye part of the test subject, and an image of the eye part of the test subject may be extracted from the image captured by the image capturing means to determine the eye closure state of the test subject from the image of the eye part.

In the breathalyzer test device according to the aspect described above, the breathalyzer test means may perform the breathalyzer test on the test subject when the vigilance detecting means determines that the eyes of the test subject have been closed continuously for at least a fixed time period.

The breathalyzer test device according to the aspect described above may also include transmitting means for transmitting a test result generated by the vigilance detecting means to a third party, and when the test subject determines that a result of the breathalyzer test is an erroneous detection, the third party may perform the breathalyzer test on the test subject based on the test result generated by the vigilance detecting means.

In the breathalyzer test device according to the aspect described above, the vigilance detecting means may detect the vigilance of the test subject at fixed time intervals.

The breathalyzer test device according to the aspect described above may also include breathalyzer test requesting means for issuing a breathalyzer test request to the test subject, and when the test subject does not take the breathalyzer test for at least a fixed time period following the test request issued by the breathalyzer test requesting means, the breathalyzer test requesting means may issue another breathalyzer test request to the test subject.

In the breathalyzer test device according to the aspect described above, the breathalyzer test device may be installed in a vehicle.

In the breathalyzer test device according to the aspect described above, the breathalyzer test device may cooperate with a drunk driving suppression device such that when it is determined that the subject is driving drunk as a result of the breathalyzer test of the breathalyzer test device, the drunk driving suppression device immobilizes the vehicle.

By taking vigilance into consideration during a breathalyzer test, the present invention may reduce the troublesomeness of the breathalyzer test.

### BRIEF DESCRIPTION OF THE DRAWINGS

The foregoing and/or further objects, features and advantages of the invention will become more apparent from the following description of the example embodiment with reference to the accompanying drawings, in which like numerals are used to represent like elements and wherein:
FIG. 1 is a constitutional diagram of a drunk driving suppression device according to an embodiment of the present invention;
FIG. 2 is a flowchart showing the flow of an operation performed at the start of driving in a vehicle in which the drunk driving suppression device shown in FIG. 1 is installed;
FIG. 3 is a flowchart showing the flow of an operation performed during driving in the vehicle in which the drunk driving suppression device shown in FIG. 1 is installed;
FIG. 4 is a flowchart showing the flow of an operation performed when a driver drives the vehicle in which the drunk driving suppression device shown in FIG. 1 is installed for the first time; and
FIG. 5 is a flowchart showing the flow of an operation performed when the driver drives the vehicle in which the drunk driving suppression device shown in FIG. 1 is installed for a second time onward.

### DETAILED DESCRIPTION OF EMBODIMENTS

An embodiment of the breathalyzer test device according to the present invention will be described below with reference to the drawings.

In this embodiment, the breathalyzer test device according to the present invention is applied to a drunk driving suppression device installed in a vehicle. The drunk driving suppression device according to this embodiment implements a breathalyzer test on a driver when testing is necessary, and immobilizes the vehicle when the driver has consumed alcohol. Further, the drunk driving suppression device according to this embodiment uses a "HELPNET" system, for example, and is capable of immobilizing the vehicle and releasing the immobilization via HELPNET.

The HELPNET system is a system for performing various types of support in relation to vehicles (passengers), such as reporting emergencies and so on, so that information may be exchanged through communication between a HELPNET center and the vehicle (transmitting means). With HELPNET, telephone conversations may be conducted between a vehicle passenger and an operator in the HELPNET center, and signals may be exchanged between the HELPNET center and various devices of the vehicle. When HELPNET is used, a contract to use HELPNET is signed in advance with each vehicle, and only contracted vehicles may connect to the HELPNET center. Appropriate examples of the operators in the HELPNET center include dispatchers of trucks, buses, taxis, and so on, police-related persons, and selected persons who have been assigned responsibility in the case of individual contracts. Note that in the drunk driving suppression device according to this embodiment, immobilization and immobilization release may be performed on the vehicle by any support center capable of exchanging information with the vehicle through communication, and these operations are not limited to the HELPNET system.

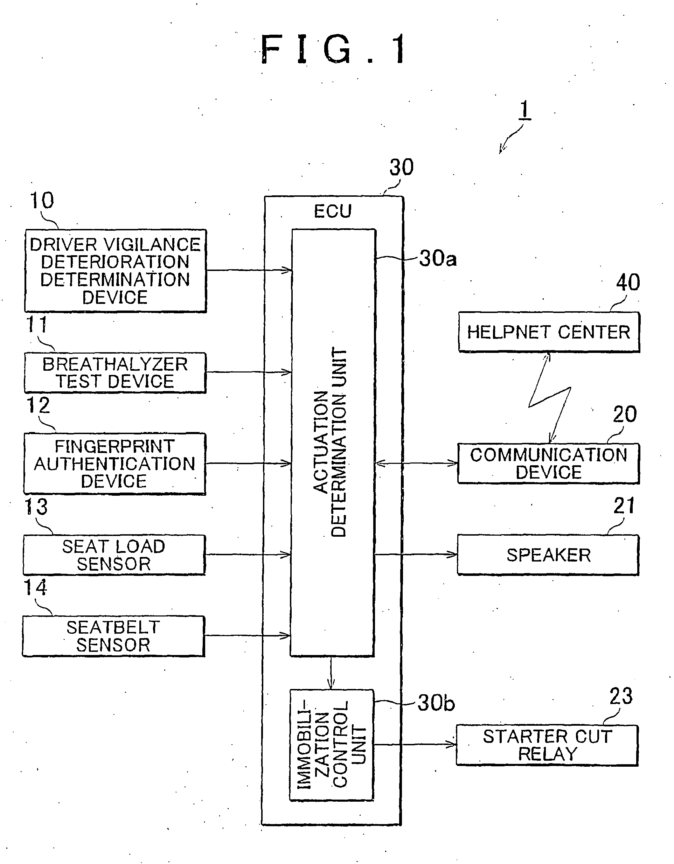

Referring to FIG 1, a drunk driving suppression device 1 according to this embodiment will be described. FIG. 1 is a constitutional diagram of the drunk driving suppression device according to this embodiment. Note that the HELPNET system is used as an emergency notification service company in this embodiment.

The drunk driving suppression device 1 suppresses drunk driving by issuing a breathalyzer test request to a driver (breathalyzer test requesting means), but reduces the troublesomeness of taking the breathalyzer test to a minimum. To achieve this, the drunk driving suppression device 1 implements the breathalyzer test only when the vigilance of the driver has deteriorated. Furthermore, the drunk driving suppression device 1 does not implement the breathalyzer test on a driver acknowledged to be a sober driver through HELPNET.

The drunk driving suppression device 1 includes a driver vigilance deterioration determination device 10, a breathalyzer test device 11, a fingerprint authentication device 12, a seat load sensor 13, a seatbelt sensor 14, a communication device 20, a speaker 21, a starter cut relay 23, and an Electronic Control Unit (ECU) 30. Further, the drunk driving suppression device 1 is capable of connecting to a HELPNET center (emergency notification service company) 40 to use HELPNET.

In this embodiment, the driver vigilance deterioration determination device 10 may be considered as vigilance detecting means according to the present invention, processing performed by the breathalyzer test device 11 and the ECU 30 may be considered as breathalyzer test means according to the present invention, and processing performed by the ECU 30 may be considered as implementation determining means according to the present invention.

The driver vigilance deterioration determination device 10 determines whether or not the vigilance of the driver has deteriorated. In the driver vigilance deterioration determination device 10, an image of the face of the driver is captured by a camera, an image of the eye part of the driver is extracted from the captured image, and a determination as to whether or not the eyes are closed (i.e. whether or not vigilance has deteriorated) is made from the eye part image. Further, an eye closure state determination result is transmitted from the driver vigilance deterioration determination device 10 to the ECU 30 at fixed time intervals. As the vigilance of the driver decreases (as sleepiness becomes more intense), the eyes gradually close until the driver falls asleep.

The breathalyzer test device 11 tests whether the driver is under the influence of alcohol. In the breathalyzer test device 11, an alcohol concentration detection sensor, such as a solid state sensor, an electrochemical sensor, a chemical reaction sensor, or an optical sensor, for example, measures alcohol concentration from the breath of the driver and determines whether or not the alcohol concentration is equal to or greater than a threshold. The threshold is used to determine whether or not the driver has consumed alcohol, and is set at a value determined in advance through experiment and the like. An alcohol consumption state determination result is then transmitted from the breathalyzer test device 11 to the ECU 30. Note that the breathalyzer test device 11 becomes capable of implementing a breathalyzer test on the driver after being activated by the ECU 30.

The fingerprint authentication device 12 authenticates the fingerprints of the driver. The fingerprint authentication device 12 reads the fingerprints of the driver using a scanner provided in the steering wheel or the like, compares the read fingerprints with pre-registered fingerprints, and identifies the driver as a registered driver when the finger prints match. Information regarding the read fingerprints and a comparison result (authentication result) are transmitted from the fingerprint authentication device 12 to the ECU 30. Note that a simple fingerprint scanning device for reading the fingerprints of the driver may be provided in place of the fingerprint authentication device.

The seat load sensor 13 is provided in each seat to detect a load applied to the seat. The seat load sensor 13 detects the load applied to the seat and transmits a detection result to the ECU 30.

The seatbelt sensor 14 is provided in each seat to detect whether or not the seatbelt is fastened. The seatbelt sensor 14 detects whether or not the seatbelt is fastened and transmits a detection result to the ECU 30.

The communication device 20 performs wireless communication with the vehicle exterior. When the driver requests connection to HELPNET by pressing a HELPNET button (not shown) or the like, or connection is requested from various device installed in the vehicle, wireless communication with the HELPNET center 40 is established via the communication device 20. During this communication, voice signals and control signals for realizing a telephone conversation, command signals, and so on are exchanged. Note that the communication device 20 may employ a cellular telephone.

The speaker 21 is provided in the vehicle and shared with other devices. When a voice signal (electric signal) is transmitted from the ECU 30, the voice signal is converted into mechanical vibration and output through the speaker 21 as speech.

The starter cut relay 23 is a relay for cutting a starter line. When the starter cut relay 23 is switched OFF, it becomes impossible to rotate a self-starting motor, and as a result, the engine may no longer be started. When a relay OFF signal from the ECU 30 is received by the starter cut relay 23, the relay is switched OFF (the relay is opened). When a relay ON signal from the ECU 30 is received by the starter cut relay 23, the relay is switched ON (the relay is closed).

The ECU 30 is constituted by a Central Processing Unit (CPU), a Read-Only Memory (ROM), a Random Access Memory (RAM) and so on, and performs overall control of the drunk driving suppression device 1. The ECU 30 functions as an actuation determination unit 30a and an immobilization control unit 30b when an application program stored in the ROM is loaded onto the RAM and executed by the CPU.

The actuation determination unit 30a issues various commands and performs various types of control based on a comprehensive determination relating to information from the respective devices 10, 11, 12, the detection results of the respective sensors 13, 14, commands from the driver, commands from the HELPNET center 40, and so on. The actuation determination unit 30a executes different applications at the start of driving, during driving, when the driver drives the vehicle for the first time, and when the driver drives the vehicle for a second time onward.

The start of driving will now be described. The actuation determination unit 30a activates the breathalyzer test device 11. The actuation determination unit 30a then receives a test result relating to a breathalyzer test performed on the driver from the breathalyzer test device 11, and when it is determined that the driver has consumed alcohol, the actuation determination unit 30a issues a command to the immobilization control unit 30b to immobilize the vehicle. Note that at the start of driving, the driver may be urged to take the breathalyzer test through the speaker 21, or the driver may take the breathalyzer test voluntarily (as a condition for starting the engine, for example).

When the driver determines at this time that the vehicle has been immobilized due to an erroneous alcohol consumption detection by the breathalyzer test device 11, s/he requests connection to HELPNET by pressing the HELPNET button or the like. In accordance with this request, the actuation determination unit 30a connects to the HELPNET center 40 via the communication device 20 so that the driver may speak with an operator in the HELPNET center 40.

Here, the driver explains to the operator that the vehicle has been immobilized due to an erroneous alcohol consumption detection, and requests release of the immobilization. The operator performs various tests and checks to determine whether or not the driver has actually consumed alcohol. To perform such tests and checks, a determination is made as to whether or not the driver has consumed alcohol, using the manner of the conversation with the driver, the vigilance determination result of the driver vigilance deterioration determination device 10, and/or the image of the face of the driver captured by the camera in the driver vigilance deterioration determination device 10, for example. When the operator determines that the driver has not consumed alcohol (i.e. that the detection of the breathalyzer test device 11 is erroneous), s/he transmits a command signal to the ECU 30 to release the immobilization of the vehicle. Upon reception of this signal, the actuation determination unit 30a issues a command to the immobilization control unit 30b to release the immobilization of the vehicle.. Alternatively, the immobilization control unit 30b receives the signal directly and releases the immobilization.

A case in which driving is underway will now be described. When the eyes of the driver are determined to be closed (vigilance is determined to have deteriorated) based on the determination result transmitted by the driver vigilance deterioration determination device 10 at fixed time intervals, the actuation determination unit 30a determines whether or not this state has continued for a first determination period or longer. The first determination period is a time period for determining that the vigilance of the driver has definitely deteriorated, and is set in advance through experiment or the like. When the eyes of the driver have been closed continuously for the first determination period or longer, the actuation determination unit 30a transmits a voice signal to the speaker 21, which is output as a voice message requesting the driver to take a breathalyzer test, and activates the breathalyzer test device 11. The message may be "please take a breathalyzer test", for example.

Incidentally, vigilance typically deteriorates following alcohol consumption. When vigilance is high, therefore, it is highly unlikely that alcohol has been consumed, and therefore, there is no need to perform a breathalyzer test. Conversely, when vigilance deteriorates below normal levels, alcohol may have been consumed, and therefore it becomes necessary to perform a breathalyzer test. Moreover, since the breathalyzer test is performed when vigilance has deteriorated, the accuracy of the test may be improved and erroneous detections may be suppressed.

After issuing the breathalyzer test request, the actuation determination unit 30a waits to receive a test result of the breathalyzer test from the breathalyzer test device 11. While waiting to receive the test result (i.e. while waiting for the driver to take the breathalyzer test), the actuation determination unit 30a determines whether or not a second determination period has elapsed since the breathalyzer test request. The second determination period is a time period for determining that the driver will not take a breathalyzer test in response to a breathalyzer test request, and is set in advance through experiment or the like. When the second determination period has elapsed following the breathalyzer test request, the actuation determination unit 30a connects to the HELPNET center 40 via the communication device 20 to request a breathalyzer test via HELPNET, thereby enabling conversation between the driver and the operator in the HELPNET center 40.

Upon connection, the operator checks the alcohol consumption condition of the driver and asks the driver to take a breathalyzer test. When the breathalyzer test is not taken in response to this breathalyzer test request, it is possible that the driver has consumed alcohol, and therefore the actuation determination unit 30a issues a command to the immobilization control unit 30b to immobilize the vehicle.. At this time, a command signal may be transmitted from the HELPNET center 40 side to immobilize the vehicle, or a determination as to whether or not to immobilize the vehicle may be made on the ECU 30 side based on whether or not the breathalyzer test has been implemented.

When the breathalyzer test is taken in accordance with the breathalyzer test request (when a test result relating to the breathalyzer test is received from the breathalyzer test device 11), the actuation determination unit 30a performs similar processing to the processing performed at the start of driving.

A case in which the driver drives the vehicle for the first time will now be described. On the basis of the detection result from the seat load sensor 13 and the detection result from the seatbelt sensor 14, the actuation determination unit 30a determines whether or not the driver is alone in the vehicle. This determination is made by determining whether or not a load is applied to a seat other than the driving seat and whether or not a seatbelt of a seat other than the driving seat is fastened. When the driver is alone in the vehicle, no person who might take the breathalyzer test in place of the driver is present in the vehicle, and it may therefore be assumed reliably that the person taking the breathalyzer test is the driver.

When it is determined that the driver is alone in the vehicle, the actuation determination unit 30a activates the fingerprint authentication device 12 and the breathalyzer test device 11 to receive information relating to the fingerprints of the driver from the fingerprint authentication device 12 and the test result of the breathalyzer test taken by the driver from the breathalyzer test device 11. When it is determined that the driver has consumed alcohol, the actuation determination unit 30a issues a command to the immobilization control unit 30b to immobilize the vehicle. Note that the trigger to start the breathalyzer test at this time is arbitrary, and may involve urging the driver to take the breathalyzer test through the speaker 21, for example.

When the driver determines that alcohol consumption has been detected erroneously at this time, s/he requests connection to HELPNET, similarly to the case described above. In accordance with this request, the actuation determination unit 30a connects to the HELPNET center 40. Here, the driver asks the operator to release the immobilization, similarly to the case described above, and the operator subjects the driver to various tests and checks, similarly to the case described above.

When it is determined that the driver has not consumed alcohol (i.e. that the breathalyzer test device 11 has detected alcohol consumption erroneously), the operator identifies the driver as a sober driver. The HELPNET center 40 then obtains the fingerprint information of the driver from the ECU 30 and registers the driver as a sober driver using the fingerprints as personal information. When the driver is registered as a sober driver, the driver is exempted, via HELPNET, from the breathalyzer test when driving alone from the next time s/he drives the vehicle onward. Furthermore, similarly to the case described above, the operator transmits a command signal for releasing the immobilization of the vehicle to the ECU 30. Upon reception of the signal, the ECU 30 releases the immobilization similarly to the case described above. The operator also transmits a command signal to the ECU 30 to switch the breathalyzer test OFF at all times during driving. When this signal is received, the actuation determination unit 30a no longer implements the breathalyzer test on the driver (no longer activates the breathalyzer test device 11) during driving.

A case in which the driver drives the vehicle for a second time onward will now be described. Similarly to the case described above, the actuation determination unit 30a determines whether or not the driver is alone in the vehicle from the seat load and the fastening state of the seatbelts. When the driver is alone, the actuation determination unit 30a connects to the HELPNET center 40 via the communication device 20. The actuation determination unit 30a also activates the fingerprint authentication device 12 to receive the fingerprint information of the driver from the fingerprint authentication device 12.

At this time, the HELPNET center 40 receives the fingerprint information of the driver from the ECU 30, and determines whether or not the driver having these fingerprints is registered as a sober driver. When the driver is registered as a sober driver, the HELPNET center 40 transmits a command signal to the ECU 30 to switch the breathalyzer test OFF at all times during driving. When this signal is received, the actuation determination unit 30a no longer implements the breathalyzer test on the driver during driving.

When immobilization control unit 30b receives a command to immobilize the vehicle from the actuation determination unit 30a or the HELPNET center 40, the immobilization control unit 30b transmits a relay OFF signal to the starter cut relay 23. When the immobilization control unit 30b receives a command to release the immobilization of the vehicle from the actuation determination unit 30a or the HELPNET center 40, the immobilization control unit 30b transmits a relay ON signal to the starter cut relay 23.

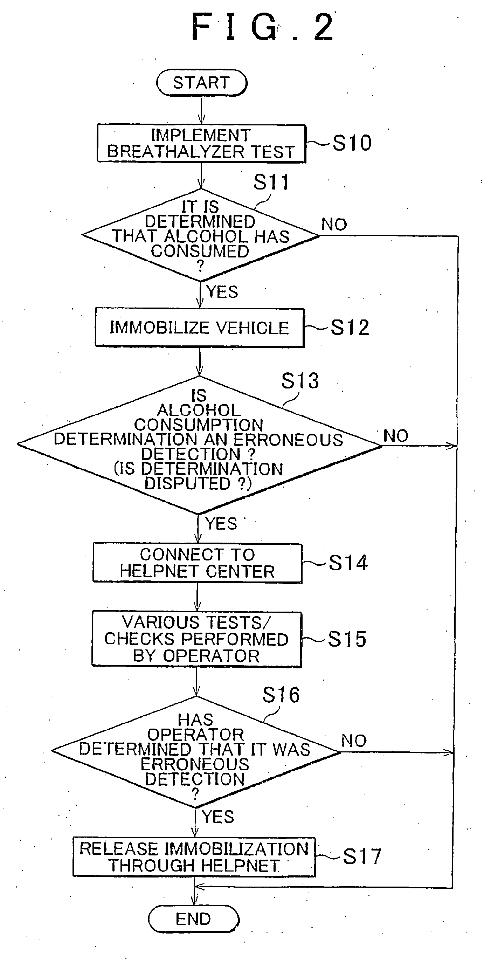

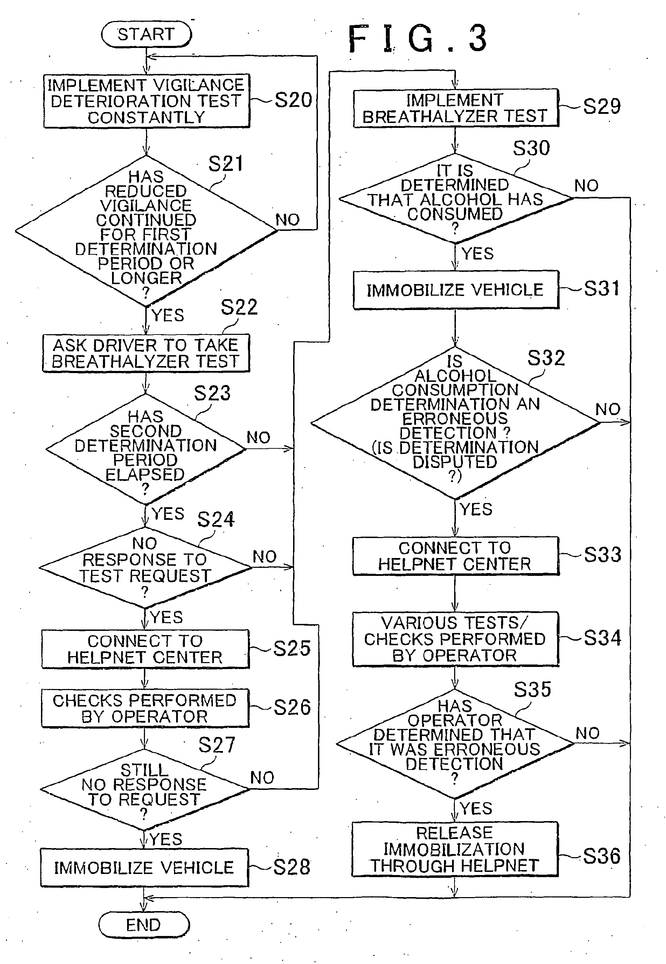

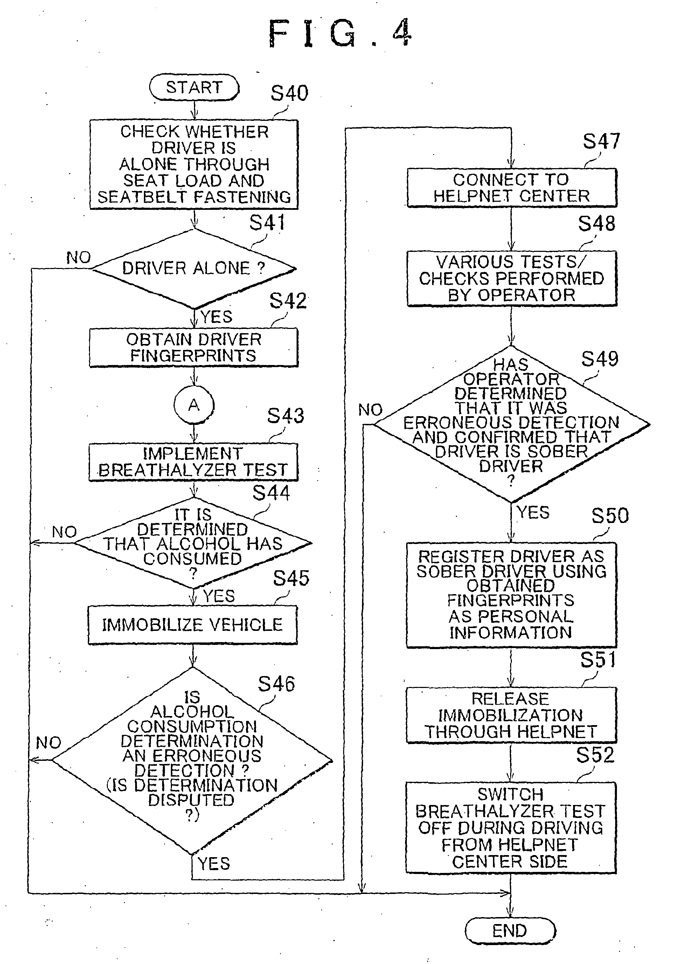

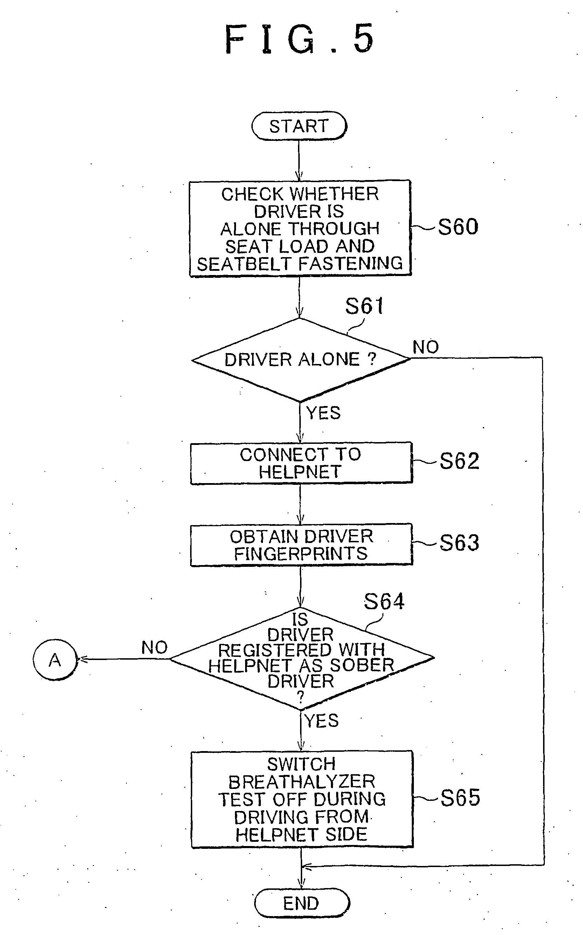

Referring to FIG. 1, operations of the drunk driving suppression device 1, the driver, and the HELPNET center 40 (i.e. the operator) will be described. Here, an operation performed at the start of driving will be described in accordance with a flowchart shown in FIG. 2, an operation performed during driving will be described in accordance with a flowchart shown in FIG. 3, an operation performed during driving for the first time will be described in accordance with a flowchart shown in FIG. 4, and an operation performed during driving for a second time onward will be described in accordance with a flowchart shown in FIG. 5. FIG. 2 is a flowchart showing the flow of an operation performed at the start of driving in the vehicle in which the drunk driving suppression device shown in FIG. 1 is installed. FIG. 3 is a flowchart showing the flow of an operation performed during driving in the vehicle in which the drunk driving suppression device shown in FIG. 1 is installed. FIG. 4 is a flowchart showing the flow of an operation performed when the driver drives the vehicle in which the drunk driving suppression device shown in FIG. 1 is installed for the first time. FIG. 5 is a flowchart showing the flow of an operation performed when the driver drives the vehicle in which the drunk driving suppression device shown in FIG. 1 is installed for a second time onward.

At the start of driving, the ECU 30 activates the breathalyzer test device 11. The breathalyzer test is implemented by having the driver blow into the breathalyzer test device 11 (S10). The breathalyzer test device 11 measures alcohol concentration from the breath of the driver, and determines whether or not alcohol has been consumed based on the alcohol concentration (S11). The breathalyzer test device 11 then transmits the result of the determination as to whether or not alcohol has been consumed to the ECU 30. When the ECU 30 receives the determination result, the ECU 30 terminates the processing when it is determined in S11 that alcohol has not been consumed.

When it is determined in S11 that alcohol has been consumed, the ECU 30 transmits a relay OFF signal to the starter cut relay 23 (S12). When the starter cut relay23 receives the relay OFF signal, the starter cut relay 23 switches the relay OFF, thereby immobilizing the vehicle (S12).

When alcohol has actually been consumed (i.e. when the alcohol consumption determination is not an erroneous detection), the driver, having become aware that s/he is under the influence of alcohol, abandons driving and alights from the vehicle (S13). As a result, drunk driving is prevented.

When alcohol has not actually been consumed, on the other hand, the driver determines that the alcohol consumption determination is an erroneous detection (S13). Therefore, the driver requests connection to HELPNET by pressing the HELPNET button or the like (S14). In accordance with this request, the ECU 30 connects to the HELPNET center 40 via the communication device 20 (S14).

When the connection to the HELPNET center 40 is established, the driver explains that the vehicle has been immobilized due to an erroneous detection of alcohol consumption. The operator conducts various tests and checks to determine whether or not the driver has consumed alcohol (S15), thereby determining whether or not an erroneous detection of alcohol consumption has occurred (S16). When it is determined in S16 that an erroneous detection has not occurred (i.e. when it is confirmed that the driver has consumed alcohol), the operator informs the driver of this determination and terminates the connection. The driver then abandons driving and alights from the vehicle.

When it is determined that an erroneous detection has occurred in S16, on the other hand (i.e. when it is confirmed that the driver has not consumed alcohol), the HELPNET center 40 transmits a command signal for releasing the immobilization of the vehicle to the ECU 30 (S17). Upon reception of this signal, the ECU 30 transmits a relay ON signal to the starter cut relay 23 (S17). Upon reception of the relay ON signal, the starter cut relay 23 switches the relay ON (S17). As a result, it becomes possible to start the engine. The driver starts the engine and begins to drive.

During driving, the driver vigilance deterioration determination device 10 continuously determines, at fixed time intervals, whether or not the vigilance of the driver has deteriorated based on the open/closed state of the eyes of the driver, and transmits the determination result to the ECU 30 (S20). Upon reception of this determination result, the ECU 30 determines whether or not a state of reduced vigilance has continued for the first determination period or longer (S21). When it is determined in S21 that the state of reduced vigilance has not continued for the first determination period or longer (when either vigilance is high or vigilance has deteriorated but this state has not yet continued for the first determination period or longer), the routine returns to S20.

When it is determined in S21 that the state of reduced vigilance has continued for the first determination period or longer, on the other hand, the ECU 30 transmits a voice signal containing a message requesting the driver to take a breathalyzer test to the speaker 21, and activates the breathalyzer test device 11 (S22). Upon reception of this voice signal, the speaker 21 outputs the voice message requesting the driver to take a breathalyzer test (S22).

After issuing the breathalyzer test request, the ECU 30 waits to receive the test result of the breathalyzer test from the breathalyzer test device 11, and determines whether or not the second determination period has elapsed since the breathalyzer test request was issued (S23). When it is determined in S23 that the second determination period has elapsed, the ECU 30 determines whether or not the driver has responded to the breathalyzer test request (S24). When it is determined in S24 that the driver has not responded to the request, the ECU 30 connects to the HELPNET center 40 via the communication device 20 (S25). When the connection is established, the ECU 30 transmits a message explaining that the driver has not responded to the breathalyzer test request or the like to the HELPNET center 40. The operator then checks the alcohol consumption condition of the driver and asks the driver to take a breathalyzer test (S26). The ECU 30 then determines whether or not the driver has responded to this breathalyzer test request (S27). When it is determined in S27 that the driver has not responded to the request, the ECU 30 transmits a relay OFF signal to the starter cut relay 23 (S28). Upon reception of the relay OFF signal, the starter cut relay 23 switches the relay OFF, thereby immobilizing the vehicle (S28). The driver, having become aware that s/he is under the influence of alcohol, abandons driving and alights from the vehicle. As a result, drunk driving is prevented.

On the other hand, when the driver takes a breathalyzer test before the second determination period elapses in response to the request issued by the ECU 30 or takes a breathalyzer test in response to the request from the operator (S29), operations of S30 to S36 (which are similar to the operations performed in S11 to S17 of FIG. 2 at the start of driving) are performed by the drunk driving suppression device 1, the driver, and the HELPNET center 40.

When the driver drives the vehicle for the first time, the ECU 30 receives a detection signal from the seat load sensor 13 and a detection signal from the seatbelt sensor 14. The ECU 30 then checks whether or not the driver is alone in the vehicle based on the load applied to each seat and the fastening state of the seatbelts (S40), thereby determining whether or not the driver is alone (S41). When it is determined in S41 that the driver is not alone in the vehicle, the ECU 30 terminates the processing.

When it is determined in S41 that the driver is alone in the vehicle, on the other hand, the ECU 30 activates the fingerprint authentication device 12. The fingerprint authentication device 12 obtains the fingerprints of the driver and transmits fingerprint information to the ECU 30 (S42). The ECU 30 receives the fingerprint information of the driver from the fingerprint authentication device 12. The ECU 30 then activates the breathalyzer test device 11. At this time, a breathalyzer test is implemented by having the driver blow into the breathalyzer test device 11 (S43). The breathalyzer test device 11 measures alcohol concentration from the breath of the driver, and determines whether or not alcohol has been consumed based on the alcohol concentration (S44). The ECU 30 receives the test result of the breathalyzer test performed on the driver from the breathalyzer test device 11. When it is determined in S44 that alcohol has been consumed, the ECU 30 transmits a relay OFF signal to the starter cut relay 23 (S45). Upon reception of the relay OFF signal, the starter cut relay 23 switches the relay OFF thereby immobilizing the vehicle (S45).

When alcohol has actually been consumed, the driver, having become aware that s/he is under the influence of alcohol, abandons driving and alights from the vehicle (S46). As a result, drunk driving is prevented.

When alcohol has not actually been consumed, on the other hand, the driver determines that the alcohol consumption determination is an erroneous detection (S46). Therefore, the driver requests connection to HELPNET by pressing the HELPNET button or the like (S47). In accordance with this request, the ECU 30 connects to the HELPNET center 40 via the communication device 20 (S47).

When the connection to the HELPNET center 40 is established, the driver explains that the vehicle has been immobilized due to an erroneous detection of alcohol consumption. The operator conducts various tests and checks to determine whether or not the driver has consumed alcohol (S48), thereby determining whether or not an erroneous detection of alcohol consumption has occurred and whether or not the driver is a sober driver (S49). When it is determined in S49 that an erroneous detection has not occurred and that the driver is a drinker, the operator informs the driver of this determination and terminates the connection. The driver then abandons driving and alights from the vehicle.

When it is determined that an erroneous detection has occurred and that the driver is a sober driver, on the other hand, the HELPNET center 40 receives the fingerprint information of the driver from the ECU 30 and registers the driver as a sober driver using the fingerprints as personal information (S50). Further, the HELPNET center 40 transmits a command signal for releasing the immobilization of the vehicle to the ECU 30 (S51). Upon reception of this signal, the ECU 30 transmits a relay ON signal to the starter cut relay 23 (S51). Upon reception of the relay ON signal, the starter cut relay 23 switches the relay ON (S51). As a result, it becomes possible to start the engine. The driver starts the engine and begins to drive. Further, the HELPNET center 40 transmits a command signal to switch the breathalyzer test OFF at all times during driving to the ECU 30 (S52). When the ECU 30 receives this signal, the ECU 30 no longer implements the breathalyzer test on the driver during driving (S52). Accordingly, the driver no longer has to take the breathalyzer test during driving.

When the driver drives the vehicle for a second time onward, the ECU 30 checks whether or not the driver is alone in the vehicle based on the load applied to each seat and the fastening state of the seatbelts (S60), similarly to the case in which the driver drives the vehicle for the first time, thereby determining whether or not the driver is alone (S61). When it is determined in S61 that the driver is not alone in the vehicles, the ECU 30 terminates the processing.

When it is determined in S61 that the driver is alone in the vehicle, on the other hand, the ECU 30 connects to the HELPNET center 40 via the communication device 20 (S62) and activates the fingerprint authentication device 12. The fingerprint authentication device 12 obtains the fingerprints of the driver and transmits fingerprint information to the ECU 30 (S63). The ECU 30 receives the fingerprint information of the driver from the fingerprint authentication device 12. The ECU 30 then transmits the fingerprint information of the driver to the HELPNET center 40. When the HELPNET center receives the fingerprint information, the HELPNET center 40 checks whether or not the driver having the fingerprints is registered as a sober driver (S64). When it is confirmed in S64 that the driver is not registered as a sober driver, the routine jumps to "A" (the operations of S43 onward) in FIG. 4, which relates to the case in which the driver drives the vehicle for the first time.

When it is confirmed in S64 that the driver is registered as a sober driver, on the other hand, the HELPNET center 40 transmits a command signal for switching the breathalyzer test OFF at all times during driving to the ECU 30 (S65). When the ECU 30 receives this signal, the ECU 30 no longer implements the breathalyzer test on the driver during driving (S65). Accordingly, the driver no longer has to take the breathalyzer test during driving.

According to the drunk driving suppression device 1, a breathalyzer test is performed only when the vigilance of a driver has deteriorated (when the breathalyzer test is required), and therefore the burden on the driver and the troublesomeness of the breathalyzer test may be reduced. Moreover, the breathalyzer test is performed based on a reduction in vigilance when there is a possibility that alcohol has been consumed, and therefore the accuracy of the test may be improved and erroneous detections may be suppressed.

Further, according to the drunk driving suppression device 1, an immobilization may only be released through HELPNET, and therefore improper immobilization release may be prevented. Moreover, according to the drunk driving suppression device 1, when the driver does not respond to a breathalyzer test request, the alcohol consumption state may be checked through HELPNET, and if necessary, immobilization may be performed. Therefore, drunk driving may be prevented reliably even when the driver does not respond to a breathalyzer test request.

Also according to the drunk driving suppression device 1, the driver may be confirmed as a sober driver through HELPNET, and a sober driver may be exempted from taking the breathalyzer test while driving. As a result, the burden on the driver and the troublesomeness of the breathalyzer test may be reduced. Moreover, since the breathalyzer test is not implemented, erroneous detections and immobilization due to erroneous detections do not occur.

An embodiment of the present invention has been described above, but the present invention is not limited to the embodiment described above, and may be implemented in various modes.

For example, in this embodiment the breathalyzer test device according to the present invention is applied to a drunk driving suppression device installed in a vehicle, but the breathalyzer test device is not limited to installation in a vehicle, and may be applied to a breathalyzer test device itself or to any other device in which a breathalyzer test is required.

Furthermore, in this embodiment the determination as to whether or not to implement the breathalyzer test is made based on vigilance, and the driver is asked to take a breathalyzer test only when vigilance has deteriorated. However, the alcohol consumption state may be determined automatically by activating an alcohol concentration detection sensor when vigilance has deteriorated, rather than asking the driver to take a test.

Furthermore, in this embodiment vigilance is determined based on the eye closure state, but may be determined based on other information such as physiological information.

Furthermore, in this embodiment immobilization is performed using a starter cut relay, but immobilization may be performed using other means such as a shift lever.

Further, in this embodiment, the HELPNET system is used to determine an erroneous alcohol consumption detection, release an immobilization, and so on, but a support center other than HELPNET may be used as long as it is capable of exchanging information with a vehicle through communication. Alternatively, if another third party exists in relation to the driver, a support center such as HELPNET need not be used.

## Claims

1. A breathalyzer test device (1) comprising:
vigilance detecting means (10) for detecting a vigilance of a test subject; and
breathalyzer test means (11, 30) for performing a breathalyzer test on the test subject based on the vigilance detected by the vigilance detecting means (10).

2. The breathalyzer test device (1) according to claim 1, wherein:
the breathalyzer test means (11, 30) includes implementation determining means (30) for determining whether or not to perform the breathalyzer test based on the vigilance detected by the vigilance detecting means (10), and
the breathalyzer test is performed only when the implementation determining means (30) determines that the breathalyzer test is to be performed.

3. The breathalyzer test device (1) according to claim 1 or 2, wherein the vigilance detecting means (10) detects the vigilance based on an eye closure state of the test subject.

4. The breathalyzer test device (1) according to claim 3, wherein the vigilance detecting means (10) includes image capturing means for capturing an image including an eye part of the test subject, and
an image of the eye part of the test subject is extracted from the image captured by the image capturing means to determine the eye closure state of the test subject from the image of the eye part.

5. The breathalyzer test device (1) according to claim 3 or 4, wherein the breathalyzer test means (11, 30) performs the breathalyzer test on the test subject when the vigilance detecting means (10) determines that the eyes of the test subject have been closed continuously for at least a fixed time period.

6. The breathalyzer test device (1) according to any one of claims 1 to 5, further comprising:
transmitting means for transmitting a test result generated by the vigilance detecting means (10) to a third party,
wherein, when the test subject determines that a result of the breathalyzer test is an erroneous detection, the third party performs the breathalyzer test on the test subject based on the test result generated by the vigilance detecting means (10).

7. The breathalyzer test device (1) according to any one of claims 1 to 6, wherein the vigilance detecting means (10) detects the vigilance of the test subject at fixed time intervals.

8. The breathalyzer test device (1) according to any one of claims 1 to 7, further comprising:
breathalyzer test requesting means for issuing a breathalyzer test request to the test subject,
wherein, when the test subject does not take the breathalyzer test for at least a fixed time period following the test request issued by the breathalyzer test requesting means, the breathalyzer test requesting means issues another breathalyzer test request to the test subject.

9. The breathalyzer test device (1) according to any one of claims 1 to 8, wherein the breathalyzer test device (1) is installed in a vehicle.

10. The breathalyzer test device (1) according to claim 9, wherein the breathalyzer test device (1) cooperates with a drunk driving suppression device (1) such that when it is determined that the subject is driving drunk as a result of the breathalyzer test of the breathalyzer test device, the drunk driving suppression device (1) immobilizes the vehicle.

## Patentansprüche

1. Alkoholmesstestgerät (1), aufweisend:
eine Wachsamkeitserfassungseinrichtung (10) zum Erfassen einer Wachsamkeit einer Versuchsperson; und
eine Alkoholmessungs-Testeinrichtung (11, 30) zum Ausführen eines Alkoholmessungstests an der Versuchsperson basierend auf der Wachsamkeit, die durch die Wachsamkeitserfassungseinrichtung (10) erfasst wird.

2. Alkoholmesstestgerät (1) nach Anspruch 1, wobei:
die Alkoholmessungs-Testeinrichtung (11, 30) eine Durchführbarkeitsbestimmungseinrichtung (30) beinhaltet, um basierend auf der durch die Wachsamkeitserfassungseinrichtung (10) erfassten Wachsamkeit zu bestimmen, ob der Alkoholmessungstest ausgeführt werden soll oder nicht, und
der Alkoholmessungstest nur dann ausgeführt wird, wenn die Durchführbarkeitsbestimmungseinrichtung (30) bestimmt, dass der Alkoholmessungstest ausgeführt werden soll.

3. Alkoholmesstestgerät (1) nach Anspruch (1) oder (2), wobei die Wachsamkeitserfassungseinrichtung (10) die Wachsamkeit basierend auf einem Augengeschlossenheitszustand der Versuchsperson erfasst.

4. Alkoholmesstestgerät (1) nach Anspruch 3, wobei die Wachsamkeitserfassungseinrichtung (10) eine Bilderaufnahmeeinrichtung zum Aufnehmen eines Bildes beinhaltet, das einen Augenteil der Versuchsperson beinhaltet, und
ein Bild des Augenteils der Versuchsperson aus dem Bild extrahiert wird, dass durch die Bildaufnahmeeinrichtung aufgenommen worden ist, um den Augengeschlossenheitszustand der Versuchsperson anhand des Bildes des Augenteils zu bestimmen.

5. Alkoholmesstestgerät (1) nach Anspruch 3 oder 4, wobei die Alkoholmessungs-Testeinrichtung (11, 30) den Alkoholmessungstest an der Versuchsperson ausführt, wenn die Wachsamkeitserfassungseinrichtung (10) bestimmt, dass die Augen der Versuchsperson zumindest eine bestimmte Zeit lang kontinuierlich geschlossen gewesen sind.

6. Alkoholmesstestgerät (1) nach einem der Ansprüche 1 bis 5, ferner aufweisend:
eine Übertragungseinrichtung zum Übertragen eines Testergebnisses, das durch die Wachsamkeitserfassungseinrichtung (10) erzeugt worden ist, an einen Dritten,
wobei, wenn die Versuchsperson bestimmt, dass es sich bei einem Ergebnis des Alkoholmessungstests um eine fehlerhafte Erfassung handelt, der Dritte den Alkoholmessungstest an der Versuchsperson basierend auf dem Testergebnis ausführt, das durch die Wachsamkeitserfassungseinrichtung (10) erzeugt worden ist.

7. Alkoholmesstestgerät (1) nach einem der Ansprüche 1 bis 6, wobei die Wachsamkeitserfassungseinrichtung (10) die Wachsamkeit des Versuchsperson in festgelegten Zeitintervallen erfasst.

8. Alkoholmesstestgerät (1) nach einem der Ansprüche 1 bis 7, ferner aufweisend:
eine Alkoholmessungstest-Anforderungseinrichtung zum Ausgeben einer Alkoholmessungstest-Anforderung an die Versuchsperson,
wobei, wenn sich die Versuchsperson im Anschluss an die Testanforderung, die durch die Alkoholmessungstest-Anforderungseinrichtung ausgegeben wird, dem Alkoholmessungstest zumindest eine bestimmte Zeit lang nicht unterzieht, die Alkoholmessungstest-Anforderungseinrichtung eine weitere Alkoholtestanforderung an die Versuchsperson ausgibt.

9. Alkoholmesstestgerät (1) nach einem der Ansprüche 1 bis 8, wobei das Alkoholmesstestgerät (1) in einem Fahrzeug eingebaut ist.

10. Alkoholmesstestgerät (1) nach Anspruch 9, wobei das Alkoholmesstestgerät (1) mit einer Vorrichtung (1) zum Verhindern von Trunkenheit am Steuer kooperiert, so dass, wenn aufgrund des Alkoholmessungstests des Alkoholmesstestgeräts bestimmt wird, dass die Versuchsperson unter Alkoholeinfluss fährt, das Fahrzeug durch die Vorrichtung zum Verhindern von Trunkenheit am Steuer fahrunfähig gemacht wird.

## Revendications

1. Dispositif d'alcootest (1) comprenant :
un moyen (10) de détection de vigilance pour détecter la vigilance d'un sujet test ; et
un moyen d'alcootest (11, 30) pour effectuer un alcootest sur le sujet test sur la base de la vigilance détectée par le moyen (10) de détection de vigilance.

2. Dispositif d'alcootest (1) selon la revendication 1, où :
le moyen d'alcootest (11, 30) comporte un moyen (30) de détermination de mise en oeuvre pour déterminer si l'on doit effectuer ou non l'alcootest sur la base de la vigilance détectée par le moyen (10) de détection de vigilance, et
l'alcootest n'est effectué que lorsque le moyen (30) de détermination de mise en oeuvre détermine que l'alcootest doit être effectué.

3. Dispositif d'alcootest (1) selon la revendication 1 ou 2, où le moyen (10) de détection de vigilance détecte la vigilance sur la base d'un état de fermeture des yeux du sujet test.

4. Dispositif d'alcootest (1) selon la revendication 3, où le moyen (10) de détection de vigilance comporte un moyen de capture d'images pour capturer une image comportant une partie des yeux du sujet test, et
une image de la partie des yeux du sujet test est extraite de l'image capturée par le moyen de capture d'image pour déterminer l'état de fermeture des yeux du sujet test à partir de l'image de la partie des yeux.

5. Dispositif d'alcootest (1) selon la revendication 3 ou 4, où le moyen d'alcootest (11, 30) effectue l'alcootest sur le sujet test lorsque le moyen (10) de détection de vigilance détermine que les yeux du sujet test ont été fermés en continu pendant au moins une période déterminée.

6. Dispositif d'alcootest (1) selon l'une quelconque des revendications 1 à 5, comprenant en outre :
un moyen de transmission pour transmettre un résultat de test généré par le moyen (10) de détection de vigilance à un tiers,
où, lorsque le sujet test détermine qu'un résultat de l'alcootest est une détection erronée, le tiers effectue l'alcootest sur le sujet test sur la base du résultat de test généré par le moyen (10) de détection de vigilance.

7. Dispositif d'alcootest (1) selon l'une quelconque des revendications 1 à 6, où le moyen (10) de détection de vigilance détecte la vigilance du sujet test à des intervalles fixes.

8. Dispositif d'alcootest (1) selon l'une quelconque des revendications 1 à 7, comprenant en outre :
un moyen demandant un alcootest pour émettre une demande d'alcootest au sujet test,
où, lorsque le sujet test ne prend pas l'alcootest pendant au moins une période déterminée suivant la demande de test émise par le moyen demandant un alcootest, le moyen demandant l'alcootest émet une autre demande d'alcootest au sujet test.

9. Dispositif d'alcootest (1) selon l'une quelconque des revendications 1 à 8, où le dispositif d'alcootest (1) est installé dans un véhicule.

10. Dispositif d'alcootest (1) selon la revendication 9, où le dispositif d'alcootest (1) coopère avec un dispositif (1) destiné à empêcher la conduite en état d'ivresse de sorte que lorsqu'on détermine que le sujet conduit en état d'ivresse à la suite de l'alcootest du dispositif d'alcootest, le dispositif (1) destiné à empêcher la conduite en état d'ivresse immobilise le véhicule.
